# DEMANDE DE BREVET EUROPEEN

(11) **EP 1 205 176 A1**
(43) Date de publication de la demande: **15.05.2002**
(21) Numéro de dépôt: 01402857.5
(22) Date de dépôt: 07.11.2001
(51) Int. Cl.: A61K 7/13

(54) **Utilisation de 2-sulphonylamino-phénols comme coupleurs en coloration d'oxydation**

(30) Priorité: 14.11.2000 FR 0014614
(71) Demandeur: L'OREAL, 75008 Paris (FR)
(72) Inventeur: Saunier, Jean-Baptiste, 75014 Paris (FR); Vidal, Laurent, 75013 Paris (FR)
(74) Mandataire: Casalonga, Axel

(57) **Abrégé**

L'invention concerne le domaine de la teinture d'oxydation des fibres kératiniques, et en particulier des fibres kératiniques humaines telles que les cheveux. Plus particulièrement, l'invention est relative à l'utilisation de certains 2-sulphonylamino-phénols de formule (I) suivante à titre de coupleur en association avec des précurseurs de colorants d'oxydation pour la teinture d'oxydation des fibres.

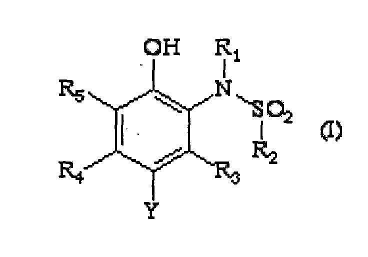

## Description

L'invention concerne le domaine de la teinture d'oxydation des fibres kératiniques, et en particulier des fibres kératiniques humaines telles que les cheveux. Plus particulièrement, l'invention est relative à l'utilisation de certains 2-sulphonylamino-phénols en association avec des précurseurs de colorants d'oxydation pour la teinture d'oxydation des fibres.

Il est connu de teindre les fibres kératiniques et en particulier les cheveux humains avec des compositions tinctoriales contenant des précurseurs de colorant d'oxydation, en particulier des paraphénylènediamines, des ortho ou para-aminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les méta-aminophénols, les métadiphénols, des naphtols non cationiques ou encore certains composés hétérocycliques tels que par exemples des coupleurs indoliques.

La variété des molécules mises en jeu au niveau des bases d'oxydation et des coupleurs, permet l'obtention d'une riche palette de couleurs.

La coloration dite "permanente", obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans la demande de brevet BE 803 712, des compositions de teinture d'oxydation contenant des 2-sulphonylamino-phénols nitrés comme colorant direct jaune ou à titre de coupleurs jaunes, en association avec des bases d'oxydation classiquement utilisées en teinture d'oxydation telles que par exemple la paraphénylènediamine, la paratoluylènediamine, la paradiméthylaminoaniline, le para-aminophénol, ou le paradiaminoanisole. De telles compositions ne sont cependant pas toujours satisfaisantes, notamment du point de vue de la puissance et de la chromaticité des colorations obtenues.

La demanderesse vient maintenant de découvrir, de façon totalement inattendue et surprenante, qu'il est possible d'obtenir de nouvelles teintures, capables de conduire à des colorations puissantes dans des nuances variant du rouge au bleu, particulièrement chromatiques et brillantes, peu sélectives, et présentant d'excellentes propriétés de résistances aux diverses agressions que peuvent subir les fibres kératiniques en associant au moins une base d'oxydation et au moins un coupleur choisi parmi certains 2-sulphonylamino-phénols.

L'invention a donc pour premier objet une composition pour la teinture d'oxydation des fibres kératiniques, et en particulier des fibres kératiniques humaines telles que les cheveux, contenant dans un milieu approprié pour la teinture desdites fibres:
- au moins une base d'oxydation;
- et au moins un coupleur choisi parmi les composés de formule (I) suivante et/ou leurs sels d'addition avec un acide : 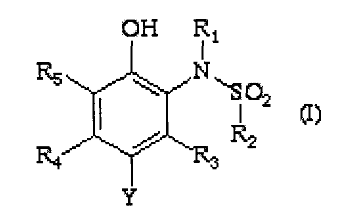
dans laquelle:
- R₁ représente un atome d'hydrogène; un radical comportant de 1 à 15 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), et dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène; ledit radical R₁ ne comportant pas de liaisons peroxyde ni de radicaux diazo, nitro et nitroso;
- R₂ représente un atome d'hydrogène; un radical comportant de 1 à 20 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), et dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène; ledit radical R₂ ne comportant pas de liaisons peroxyde ni de radicaux diazo, nitro et nitroso;
- R₃, R₄ et R₅, identiques ou différents, représentent un atome d'hydrogène ou d'halogène; un radical comportant de 1 à 20 atomes de carbone, linéaire ou ramifié (la ou les ramifications pouvant alors former un ou plusieurs cycles comportant de 3 à 7 chaînons), pouvant contenir une ou plusieurs liaisons doubles et/ou une ou plusieurs liaisons triples (lesdites liaisons doubles conduisant éventuellement à des groupements aromatiques), et dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène; ledit radical ne comportant pas de liaisons peroxydes ni de radicaux diazo, nitro et nitroso; et étant entendu que R₅ ne peut représenter un radical hydroxyle, thio ou amino; et étant entendu que les radicaux R₃, R₄ et R₅ ne peuvent être reliés au cycle benzénique de la formule (I) par une liaison - NH-NH-;
- Y représente un atome d'hydrogène ou d'halogène; un groupement -OR₆, -SR₆ ou -NH-SO₂R₆ dans lesquels R₆ représente un radical alkyle en C₁-C₆, linéaire ou ramifié (la ou les ramifications pouvant alors former un ou plusieurs cycles comportant de 3 à 6 chaînons), éventuellement substitué par un ou plusieurs radicaux choisis dans le groupe : halogène, hydroxy, alcoxy en C₁-C₄, amino, aminoalkyle en C₁-C₄; un radical phényle, éventuellement substitué par un ou deux radicaux choisis dans le groupe alkyle en C₁-C₄, trifluorométhyle, carboxy, alcoxycarbonyle en C₁-C₄, halogène, hydroxy, alcoxy en C₁-C₄, amino, aminoalkyle en C₁-C₄; un radical benzyle.

Comme indiqué précédemment, la composition de teinture d'oxydation contenant le ou les composés de formule (I) conforme à l'invention permet d'obtenir des colorations puissantes dans des nuances variant du rouge au bleu et présentant de plus une ténacité remarquable aux différents traitements que peuvent subir les fibres kératiniques. Ces propriétés sont particulièrement remarquables notamment en ce qui concerne la résistance des colorations obtenues vis-à-vis de l'action des intempéries, des lavages, de l'ondulation permanente et de la transpiration.

L'invention a également pour objet un procédé de teinture d'oxydation des fibres kératiniques mettant en oeuvre cette composition tinctoriale.

Selon l'invention, lorsque qu'il est indiqué qu'un ou plusieurs des atomes de carbone du ou des radicaux R₁ à R₅ peuvent être remplacés par un atome d'oxygène, d'azote, ou de soufre ou par un groupement SO₂ et/ou que lesdits radicaux R₁ à R₅ peuvent contenir une ou plusieurs liaisons doubles et/ou une ou plusieurs liaisons triples, cela signifie que l'on peut, à titre d'exemple, faire les transformations suivantes :

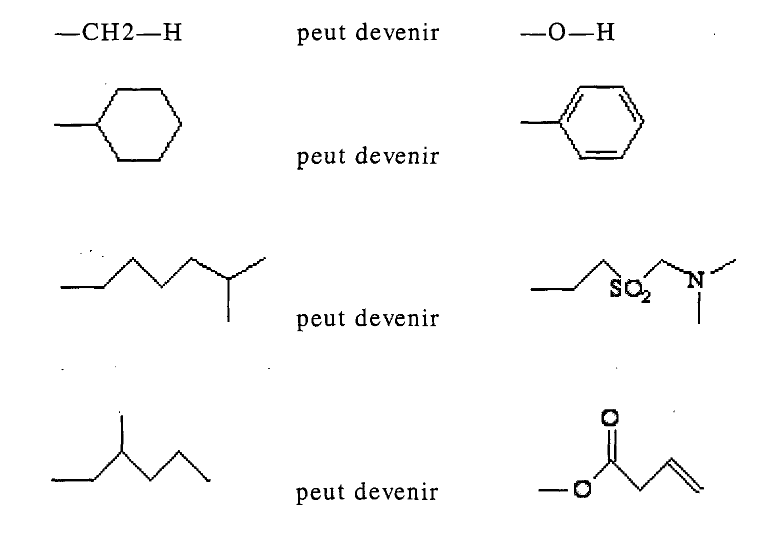

Selon l'invention, R₁ désigne de préférence un atome d'hydrogène; un groupement A₁, A₂, A₃, A₄ ou A₅, tels que définis ci-après.

Selon l'invention, on entend par groupement A₁ un radical hydrocarboné en C₁-C₈, linéaire ou ramifié, pouvant porter une ou deux doubles liaisons ou une triple liaison, être substitué ou non substitué par un groupement choisi parmi un groupement A₂, un groupement A₄, un groupement A₅, être substitué ou non substitué par un ou deux groupements, identiques ou différents, choisis parmi les groupements N-alkyl(C₁-C₃)amino, N-alkyl(C₁-C₃)-N-alkyl(C₁-C₃)amino, alcoxy(C₁-C₆), oxo, alcoxycarbonyl, acyloxy, amido, acylamino, uréyl, sulfoxy, sulfonyl, sulfonamido, sulfonylamino, bromo, cyano, carboxy, et être substitué ou non substitué par un ou plusieurs groupements hydroxyl, fluoro ou chloro.

On entend par groupement A₂, un groupement aromatique de type phényle, benzyle ou naphtyle, pouvant être substitué ou non substitué par un à trois groupements, identiques ou différents, choisis parmi les groupements méthyle, trifluorométhyle, éthyle, isopropyle, butyle, pentyle, fluoro, chloro, bromo, méthoxy, trifluorométhoxy, éthoxy, propyloxy, acétyloxy, acétyle, et cyano.

On entend par groupement A₃ des groupements hétéroaromatiques choisis parmi les groupements furanyle, thiophényle, pyrrolyle, imidazolyle, thiazolyle, oxazolyle, 1,2,3-triazolyle, 1,2,4-triazolyle, isoxazolyle, isothiazolyle, pyrazolyle, pyrazolotriazolyle, pyrazoloimidazolyle, pyrrolotriazolyle, pyrazolopyrimidyle, pyrazolopyridyle, pyridyle, pyrimidyle, benzimidazolyle, benzoxazolyle, benzothiazolyle, indolyle, indolidinyle, isoindolyle, indazolyle, benzotriazolyle, quinolinyle, benzopyrimidyle, substitués éventuellement par 1 à 3 radicaux choisis parmi les alkyle en C₁-C₄ linéaire ou ramifié, (poly)hydroxyalkyle en C₁-C₄, carboxy, alcoxycarbonyle, halogène, amido, amino, hydroxy.

On entend par groupement A₄, un cycloalkyle en C₃-C₇, un radical norbornanyle, portant éventuellement une double liaison et éventuellement substitué par 1 ou 2 radicaux définis par alkyle en C₁-C₄ linéaire ou ramifié, (poly)hydroxyalkyle en C₁-C₄, carboxy, alcoxycarbonyle, halogène, amido, amino, hydroxy.

On entend par groupement A₅ un hétérocycle défini par dihydrofuranyle, tétrahydrofuranyle, butyrolact-one-yle, dihydrothiophényle, tétrahydrothiophényle, tétrahydrothiophén-one-yle, iminothiolanyle, dihydropyrrolyle, pyrrolidinyle, pyrrolidinone-yle, imidazolidin-one-yle, imidazolidinethione-yle, oxazolidinyle, oxazolidinoneyle, oxazolanethioneyle, thiazolidinyle, isothiazol-one-yle, mercaptothiazolinyle, pyrazolidin-one-yle, dioxolanyle, pentalactone, dioxanyle, dihydropyridinyle, pipéridinyle, pentalactameyle, morpholinyle, pyrazoli(di)nyle, pyrimi(di)nyle, pyrazinyle, pipérazinyle, et azépinyle.

Parmi ces substituants, R₁ représente de préférence un atome d'hydrogène; un radical méthyle, éthyle, isopropyle, allyle, phényle, benzyle, fluorobenzyle, hydroxybenzyle difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle, 2-naphtométhyle; un groupement 2-hydroxéthyle, 2-méthoxyéthyle, ou 2-éthoxyéthyle.

De façon encore plus préférentielle, R₁ représente un atome d'hydrogène ou un radical méthyle.

Selon l'invention, R₂ désigne de préférence un atome d'hydrogène, un groupement amino; un groupement A₁, A₂, A₃, A₄ ou A₅ tels que définis précédemment, éventuellement séparés du soufre (en position 8) de la fonction sulfonamide du composé de formule (I) par un groupement -NH, ou -N-alkyl(C₁-C₃)-.

Parmi ces substituants, R₂ désigne de préférence un radical choisi dans le groupe (G1) constitué par un radical méthyle, trifluorométhyle, éthyle, 2-chloroéthyle, propyle, 3-chloropropyle, isopropyle, butyle, phényle, éthoxy, amino et diméthylamino.

De façon encore plus préférentielle, R₂ représente un radical méthyle, éthyle, phényle ou diméthylamino.

Selon l'invention, R₃ et R₄, identiques ou différents, désignent de préférence un atome d'hydrogène ou d'halogène; un groupement hydroxyle ou amino; un groupement A₁, A₄, ou A₅ tels que définis précédemment; un groupement A₁, A₂, A₃, A₄ ou A₅ tels que définis précédemment et séparés du noyau phénolique de la formule (I) par un atome d'oxygène ou par un groupement -NH-, -N-alkyl(C₁-C₃)-, O(CO)-, -NH(CO)-, -N-alkyl(C₁-C₃)(CO)-, -NH[C=NH]-, -NH(CO)NH-, -NH(CO)N-alkyl(C₁-C₃)-, -NH(CO)O-, -NHSO₂-, -NHSO₂NH-, ou -NHSO₂N-alkyl(C,-C₃)-.

Parmi ces substituants, R₃ représente de préférence un atome d'hydrogène ou de chlore; un radical méthyle, hydroxyméthyle, méthoxyméthyle, 1-hydroxyéthyle, aminométhyle, méthylaminométhyle; un radical hydroxy, méthoxy, acétoxy; un radical amino, méthylamino, 2-hydroxyéthylamino; un groupement -NH(CO)R₇ dans lequel R₇ représente un des radicaux énumérés dans le groupe (G2) constitué par les radicaux méthyle, éthyle, propyle, isopropyle, butyle, isobutyle, tert-butyle, pentyle, isopentyle, néopentyle, hexyle; cyclopropyle, cyclobutyle, cyclopentyle, cyclopentylméthyle, 3-cyclopentyl-propyle, cyclohexyle, 2-cyclohexyléthyle, norbornan-2-yle, vinyle, 1-méthylvinyle, 2-méthylvinyle, 2,2-diméthylvinyle, allyle, 3-butenyle; phényle, méthylphényle, diméthylphényle, 2,4,6-triméthylphényle, 4-éthylphényle, (trifluorométhyl)phényle, hydroxyphényle, méthoxyphényle, éthoxyphényle, acétoxyphényle, (trifluorométhoxy)phényle, aminophényle, 4-diméthylaminophényle, fluorophényle, difluorophényle, fluoro(trifluorométhyl)phényle, chlorophényle, dichlorophényle, bromophényle, napht1-yle, napht-2-yle, (2-méthoxy)napht-1-yl, benzyle, 4'-méthoxybenzyle, 2',5'diméthoxybenzyle, 3',4'-diméthoxy-benzyle, 4'-fluorobenzyle, 4'-chlorobenzyle, phénéthyle, 2-phénylvinyle, (1-naphtyl)méthyle, (2-naphtyl)méthyle; tétrahydrofuran2-yle, furan-2-yle, 5-méthyl-2-(trifluorométhyl)furan-3-yle, 2-méthyl-5-phénylfuran-3-yle, thiophén-2-yle, (thiophén-2-yl)méthyle, 3-chlorothiophén-2-yle, 2,5-dichlorothiophén-3-yle, benzothiophén-2-yle, 3-chlorobenzothiophén-2-yl, isoxazol-5-yle, 5-méthylisoxazol-3-yle, 3,5-diméthylisoxazol-4-yl, 1,3-diméthylpyrazol-5-yle, 1-éthyl-3-méthylpyrazol-5-yle, 1-tertbutyl-3-méthylpyrazol-5-yle, 3-tertbutyl-1-méthylpyrazol-5-yle, 4-bromo-1-éthyl-3-méthylpyrazol-5-yle, indol-3-ylcarboxyle, pyridinyle, chloropyridinyle, dichloropyridinyle, 5-(bromo)pyridin-3-yle, piperazin-2-yle, quinoxal-2-yle; fluorométhyle, difluorométhyle, trifluorométhyle, 1,1,2,2-tétrafluoroéthyle, pentafluoroéthyle, heptafluoropropyle, 1,1,2,2,3,3,4,4-octafluorobutyle, nonafluorobutyle, chlorométhyle, chloroéthyle, 1,1-diméthyl-2-chloroéthyle, 1,2-dichloroéthyle, 1-chloropropyle, 3-chloropropyle, 4-chlorobutyle, hydroxyméthyle, méthoxyméthyle, phénoxyméthyle, (4-chlorophénoxy)méthyle, benzyloxyméthyle, acétoxyméthyle, 1,2-dihydroxyéthyle, 1-phénoxyéthyle, 1-acétoxyéthyle, 2-(2-carboxyéthoxy)éthyle, 1-phénoxyéthyle, 1-acétoxyéthyle, méthoxycarbonyle, éthoxycarbonyle, (méthoxycarbonyl)méthyle, 2-carboxyéthyle, 2-(méthoxycarbonyl)éthyle, 2-carboxycylopropyle, 2-carboxycyclohexane; méthoxy, éthoxy, propoxy, isopropoxy, butoxy, isobutoxy, pentoxy, néopentoxy, hexyloxy, cyclopentyloxy, cyclohexyloxy, vinyloxy, allyloxy, propargyloxy, chlorométhoxy, 1-chloroéthoxy, 2-méthoxyéthoxy, 4-chlorobutoxy, phénoxy, 4-méthyphénoxy, 4-fluorophénoxy, 4-bromophénoxy, 4-chlorophénoxy, 4-méthoxyphénoxy, naphth-2-yloxy, benzyloxy; amino, méthylamino, éthylamino, propylamino, isopropylamino, butylamino, cyclohexylamino, allylamino, 2-chloroéthylamino, 3-chloropropylamino, carboxyméthylamino, phénylamino, fluorophénylamino, (trifluorométhyl)phénylamino, chlorophénylamino, bromophénylamino, 4-acétylphénylamino, méthoxyphénylamino, (trifluorométhoxy)phénylamino, naphth-1-ylamino, benzylamino, phénéthylamino, pyrid-3-ylamino, diméthylamino, 1-pyrolidinyle, et 4-morpholinyle; ou un groupement -NHSO₂R₈, dans lequel R₈ représente un des radicaux énumérés dans le groupe (G1) tel que défini ci-dessus.

De façon encore plus préférentielle, R₃ représente un atome d'hydrogène; un radical méthyle, hydroxyméthyle, aminométhyle, hydroxy, méthoxy, amino, ou méthylamino; un groupement -NH(CO)R₈ dans lequel R₈ est choisi dans le groupe (G3) constitué par les radicaux méthyle, éthyle, propyle, allyle, phenyle, tétrahydrofuran-2-yl, furan-2-yl, thiophène-2-yle, pyridinyle, piperazin-2-yle, fluorométhyle, chlorométhyle, 2-chloroéthyle, méthoxyméthyle, acétoxyméthyle, 1,2-dihydroxyéthyle, méthoxycarbonyle, 2-carboxyéthyle, méthoxy, éthoxy, propoxy, allyloxy, 2-chloroéthoxy, 2-méthoxyéthoxy, amino, éthylamino, allylamino, 2-chloroéthylamino, pyridylamino, diméthylamino, 1-pyrolidinyle, et 4-morpholinyle; ou un groupement méthanesulfonylamino, éthanesulfonylamino, benzènesulfonylamino ou diméthylaminosulfonylamino.

Parmi ces substituants, R₄ représente de préférence un atome d'hydrogène ou de chlore; un radical méthyle, éthyle, hydroxyméthyle, méthoxyméthyle, aminométhyle, ou méthylaminométhyle; un groupement hydroxy, méthoxy, acétoxy, amino, méthylamino, N-pipéridino, ou N-morpholino; un groupement -NH(CO)R₁₀ dans lequel R₁₀ représente un des radicaux énumérés dans le groupe (G2) défini ci-dessus; ou un groupement -NHSO₂R₁₁ dans lequel R₁₁ représente un des radicaux énumérés dans le groupe (G1) défini ci-dessus.

De façon encore plus préférentielle, R₄ représente un atome d'hydrogène ou de chlore; un radical méthyle, hydroxyméthyle, aminométhyle, hydroxy, méthoxy, amino, ou méthylamino; un groupement -NH(CO)R₁₂ dans lequel R₁₂ représente un des radicaux énumérés dans le groupe (G3) défini ci-dessus; ou un groupement méthanesulfonylamino, éthanesulfonylamino, benzènesulfonylamino ou diméthylaminosulfonylamino.

Selon l'invention, R₅ est de préférence choisi parmi un atome d'hydrogène ou d'halogène; un groupement A₁, A₄, ou A₅ tels que définis précédemment; un groupement A₁, A₂, A₃, A₄ ou A₅ tels que définis précédemment et séparés du noyau phénolique des composés de formule (I) par un atome d'oxygène, de soufre, ou par un groupement-NH-, -N-alkyl(C₁-C₃)-, -NH(CO)-, -N-alkyl(C₁-C₃)(CO)-, -NH[C=NH]-, -NH(CO)NH-, -NH(CO)N-alkyl(C₁-C₃)-, ou -NH(CO)O-.

Parmi ces substituants, R, représente de préférence un atome d'hydrogène, de chlore, de fluor, ou de brome; un radical méthyle, trifluorométhyle, allyle, hydroxyméthyle, méthoxyméthyle, 1-hydroxyéthyle, aminométhyle, méthylaminométhyle, méthoxy, acétoxy, ou méthylamino; un groupement -NH(CO)R₁₃ dans lequel R₁₃ représente un des radicaux énumérés dans le groupe (G2) défini ci-dessus; ou un groupement -NHSO₂R₁₄ dans lequel R₁₄ représente un des radicaux énumérés dans le groupe (G1) défini ci-dessus.

De façon encore plus préférentielle, R₅ représente un atome d'hydrogène, de chlore, ou de fluor, un groupement méthyle, hydroxyméthyle, aminométhyle, méthoxy, méthylamino; un groupement -NH(CO)R₁₅ dans lequel R₁₅ représente un des radicaux énumérés dans le groupe (G3) défini ci-dessus; ou un groupement méthanesulfonylamino, éthanesulfonylamino, benzènesulfonylamino ou diméthylaminosulfonylamino.

Selon l'invention, Y est de préférence choisi parmi un atome d'hydrogène, de chlore, de fluor ou de brome; un groupement méthoxy, éthoxy, propoxy, benzyloxy, ou phénoxy; ou un groupement -OCH₂CH₂OCH₃, -OCH₂CH₂N(CH₃)₂, -OCH₂(CO)OH, -OCH₂(CO)OCH₃, -OCH₂(CO)OC₂H₅, -SCH₂CH₂CO₂H, ou -NHSO₂CH₃.

De façon encore plus préférentielle, Y est choisi parmi un atome d'hydrogène, ou de chlore; un groupement méthoxy, -OCH₂(CO)OH, ou -OCH₂(CO)OCH₃.

Parmi les composés de formule (I), on préfère particulièrement ceux dans lesquels :
i)
   - R₁ représente un atome d'hydrogène;
   - R₂ représente un radical méthyle, éthyle, phényle ou diméthylamino;
   - R₃ représente un radical hydroxy, amino, ou méthylamino; un groupement -NH(CO)R₁₆ dans lesquels R₁₆ représente un radical choisi dans le groupe (G4) constitué par les radicaux méthyle, méthoxyméthyle, 2-carboxyéthyle, méthoxy, amino, éthylamino, 1-pyrolidinyle; méthanesulfonylamino, éthanesulfonylamino, et diméthylaminosulfonylamino;
   - R₄ représente un atome d'hydrogène ou de chlore; ou un groupement méthyle;
   - R₅ représente un atome d'hydrogène, de chlore ou de fluor; ou un groupement méthyle;
   - Y représente un atome d'hydrogène ou de chlore; ou un groupement méthoxy, ou -OCH₂(CO)OCH₃;
ii)
   - R₁ représente un atome d'hydrogène;
   - R₂ représente un radical méthyle, éthyle, phényle ou diméthylamino;
   - R₃ représente un atome d'hydrogène ou un radical méthyle;
   - R₄ représente un groupement hydroxy, amino, méthylamino, ou -NH(CO)R₁₇ dans lesquels R₁₇ représente un des radicaux énumérés dans le groupe (G4) défini ci-dessus; un groupement méthanesulfonylamino, éthanesulfonylamino, ou diméthylaminosulfonylamino;
   - R₅ représente un atome d'hydrogène, de chlore ou de fluor, ou un groupement méthyle, méthoxy, ou méthylamino;
   - Y représente un atome d'hydrogène ou de chlore, ou un groupement méthoxy, ou -OCH₂(CO)OCH₃;
iii)
   - R₁ représente un atome d'hydrogène;
   - R₂ représente un radical méthyle, éthyle, phényle ou diméthylamino;
   - R₃ représente un atome d'hydrogène ou un radical méthyle;
   - R₄ représente un atome d'hydrogène ou de chlore, un radical méthyle, méthoxy, ou méthylamino;
   - R₅ représente un groupement méthylamino, ou -NH(CO)R₁₈ dans lequel R₁₈ représente un des radicaux énumérés dans le groupe (G4) défini ci-dessus; un groupement méthanesulfonylamino, éthanesulfonylamino, ou diméthylaminosulfonylamino;
   - Y représente un atome d'hydrogène ou de chlore; ou un groupement méthoxy, ou -OCH₂(CO)OCH₃;
iv)
   - R₁ représente un atome d'hydrogène;
   - R₂ représente un radical méthyle, éthyle, phényle ou diméthylamino;
   - R₃ représente un atome d'hydrogène ou un radical méthyle;
   - R₄ représente un atome d'hydrogène ou de chlore, ou un radical méthyle;
   - R₅ représente un atome d'hydrogène, de chlore ou de fluor; ou un radical méthyle;
   - Y représente un atome d'hydrogène ou de chlore, ou un groupement méthoxy, ou -OCH₂(CO)OCH₃.
Parmi les composés de formule (I) ci-dessus, on peut citer :
- le N-(2-hydroxy-phényl)-méthanesulfonamide;
- le N-(2-hydroxy-4-méthyl-phényl)-méthanesulfonamide;
- le N-(2-hydroxy-4-amino-phényl)-méthanesulfonamide;
- le N-(2-hydroxy-4-acétylamino-phényl)-méthanesulfonamide;
- le N-(2-hydroxy-4-méthoxycarbonylamino-phényl)-méthanesulfonamide;
- le N-(2-hydroxy-5-chloro-phényl)-méthanesulfonamide;
- le N-(2-hydroxy-4-méthyl-5-chloro-phényl)-méthanesulfonamide;
- le N-(2-hydroxy-4-amino-5-chloro-phényl)-méthanesulfonamide;
- le N-(2-hydroxy-4-acétylamino-5-chloro-phényl)-méthanesulfonamide;
- le N-(2-hydroxy-4-méthoxycarbonylamino-5-chloro-phényl)-méthanesulfonamide;
- le N-(2-hydroxy-5-méthoxy-phényl)-méthanesulfonamide;
- le N-(2-hydroxy-4-méthyl-5-méthoxy-phényl)-méthanesulfonamide;
- le N-(2-hydroxy-4-amino-5-méthoxy-phényl)-méthanesulfonamide;
- le N-(2-hydroxy-4-acétylamino-5-méthoxy-phényl)-méthanesulfonamide;
- le N-(2-hydroxy-4-méthoxycarbonylamino-5-méthoxy-phényl)-méthanesulfonamide;
- le N-(2-hydroxy-6-amino-phényl)-méthanesulfonamide;
- le N-(2-hydroxy-6-acétylamino-phényl)-méthanesulfonamide;
- le N-(2-hydroxy-4,6-diamino-phényl)-méthanesulfonamide;
- le N-(2-hydroxy-4-acétylamino-6-amino-phényl)-méthanesulfonamide;
- le N-(2-hydroxy-3,5-dichloro-4-méthyl-phényl)-méthanesulfonamide;
- le N-(2-hydroxy-3,5-dichloro-4-amino-phényl)-méthanesulfonamide;
- le N-(2-hydroxy-3,5-dichloro-4-acétylamino-phényl)-méthanesulfonamide;
- le N-(2-hydroxy-3,5-dichloro-4-méthoxycarbonylamino-phényl)méthanesulfonamide;
- le N-(2-hydroxy-3-méthanesulfonylamino-phényl)-méthanesulfonamide;
- le N-(2-hydroxy-phényl)-benzènesulfonamide;
- le N-(2-hydroxy-4-méthyl-phényl)-benzènesulfonamide;
- le N-(2-hydroxy-4-amino-phényl)-benzènesulfonamide;
- le N-(2-hydroxy-4-acétylamino-phényl)-benzènesulfonamide;
- le N-(2-hydroxy-4-méthoxycarbonylamino-phényl)-benzènesulfonamide;
- le N-(2-hydroxy-5-chloro-phényl)-benzènesulfonamide;
- le N-(2-hydroxy-4-méthyl-5-chloro-phényl)-benzènesulfonamide;
- le N-(2-hydroxy-4-amino-5-chloro-phényl)-benzènesulfonamide;
- le N-(2-hydroxy-4-acétylamino-5-chloro-phényl)-benzènesulfonamide;
- le N-(2-hydroxy-4-méthoxycarbonylamino-5-chloro-phényl)-benzènesulfonamide;
- le N-(2-hydroxy-5-méthoxy-phényl)-benzènesulfonamide;
- le N-(2-hydroxy-4-méthyl-5-méthoxy-phényl)-benzènesulfonamide;
- le N-(2-hydroxy-4-amino-5-méthoxy-phényl)-benzènesulfonamide;
- le N-(2-hydroxy-4-acétylamino-5-méthoxy-phényl)-benzènesulfonamide;
- le N-(2-hydroxy-4-méthoxycarbonylamino-5-méthoxy-phényl)-benzènesulfonamide;
- le N-(2-hydroxy-6-amino-phényl)-benzènesulfonamide;
- le N-(2-hydroxy-6-acétylamino-phényl)-benzènesulfonamide;
- le N-(2-hydroxy-4,6-diamino-phényl)-benzènesulfonamide;
- le N-(2-hydroxy-4-acétylamino-6-amino-phényl)-benzènesulfonamide;
- le N-(2-hydroxy-3,5-dichloro-4-méthyl-phényl)-benzènesulfonamide;
- le N-(2-hydroxy-3,5-dichloro-4-amino-phényl)-benzènesulfonamide;
- le N-(2-hydroxy-3,5-dichloro-4-acétylamino-phényl)-benzènesulfonamide;
- le N-(2-hydroxy-3,5-dichloro-4-méthoxycarbonylamino-phényl)benzènesulfonamide;
- le N-(2-hydroxy-3-benzènesulfonylamino-phényl)-benzènesulfonamide;
et leurs sels d'addition avec un acide.

Les composés de formules (I) conformes à l'invention peuvent être préparés selon des méthodes bien connues de l'état de la technique et décrites, par exemple, dans les demandes de brevets ou brevets EP 0 718 277, EP 0 576 172, US 4 250 246, DE 2 906526, US 4 200 466, US 4 004 028, US 3 920 444, DE 2 156 480, US 3 660 487.

Le ou les composés de formules (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La composition de teinture d'oxydation conforme à l'invention contient une ou plusieurs bases d'oxydation qui sont de préférence choisies parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylène diamine, la 2-chloroparaphénylènediamine, la 2,3-diméthyl paraphénylènediamine, la 2,6 diméthyl paraphénylènediamine, la 2,6 diéthyl paraphénylènediamine, la 2,5 diméthyl paraphénylènediamine, la N, N diméthyl paraphénylènediamine, la N, N diéthyl paraphénylènediamine, la N, N dipropyl paraphénylènediamine, la 4-amino N,N-diéthyl 3-méthyl aniline, la N,N-bis-(β-hydroxyethyl)paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N, N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-diéthyl N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl-aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazol 5-one, le 1-phényl 3-méthyl pyrazol 5-one, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisable dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol; le glycérol; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (Il) suivante: 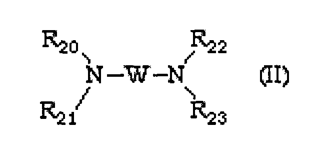 dans laquelle W est un reste propylène substitué ou non substitué par un groupement hydroxyle ou un radical alkyle en C₁-C₆; R₂₀, R₂₁, R₂₂ et R₂₃, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un procédé de teinture d'oxydation des fibres kératiniques et en particulier des fibres kératiniques humaines telles que les cheveux, mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases, les tyrosinases et les oxydoréductases parmi lesquelles on peut en particulier mentionner les pyranose oxydases, les glucose-oxydases, les glycérol-oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

L'invention a enfin pour objet un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

Chacun des essais détaillés ci-dessous correspond à l'utilisation des bases 1 à 5 suivantes et des coupleurs 1 à 4 suivants du type 2-sulphonylamino-phénol de formule (I):
Coupleur 1 : N-(2-hydroxy-4-méthyl-phényl)-méthanesulfonamide;
Coupleur 2 : N-(4-amino-2hydroxy-phényl)-méthanesulfonamide;
Coupleur 3 : N-(4-amino-2hydroxy-phényl)-benzènesulfonamide;
Coupleur 4 : N-(2-hydroxy-4-méthyl-phényl)-benzènesulfonamide.

Base 1 : paraphénylènediamine;
Base 2 : para-aminophénol
Base 3 : 4,5 diamino 1-éthyl 3-méthyl pyrazole, 2HCl
Base 4 : 3,7-diaminopyrazolo-[1,5-a]-pyrimidine, 2HCl;
Base 5 : N, N-bis hydroxyéthyl paraphénylènediamine, sulfate.

A partir de ces bases et coupleurs, on prépare des compositions tinctoriales comprenant (teneur en gramme):

| | |
|---|---|
| Coupleur | (*) |
| base | (*) |
| Alkyl (C8/C10 50/50) polyglucoside en solution aqueuse à 60% tamponnée | 5,4 g |
| Alcool éthylique 96 degrés dénaturé | 18 g |
| Alcool benzylique | 1,8 g |
| Polyéthylène glycol (8 OE) | 2,7 g |
| Acide diéthylène triamine pentacétique, sel pentasodique en solution aqueuse à 40% | 1,08 g |
| métabisulfite de sodium en poudre | 0,585 g |
| ammoniaque | 10 g |
| Eau déminéralisée | qsp 100 g |

| | |
|---|---|
| (*) les quantités de coupleur et de base utilisées sont indiquées dans les tableaux ci dessous. | |

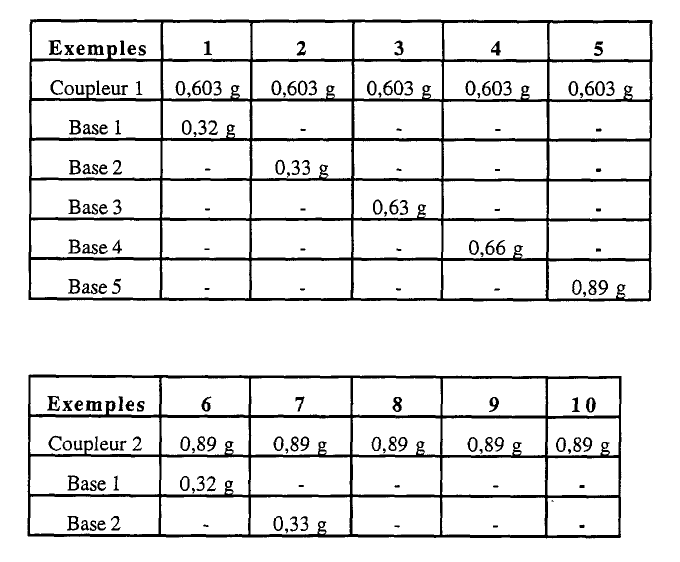

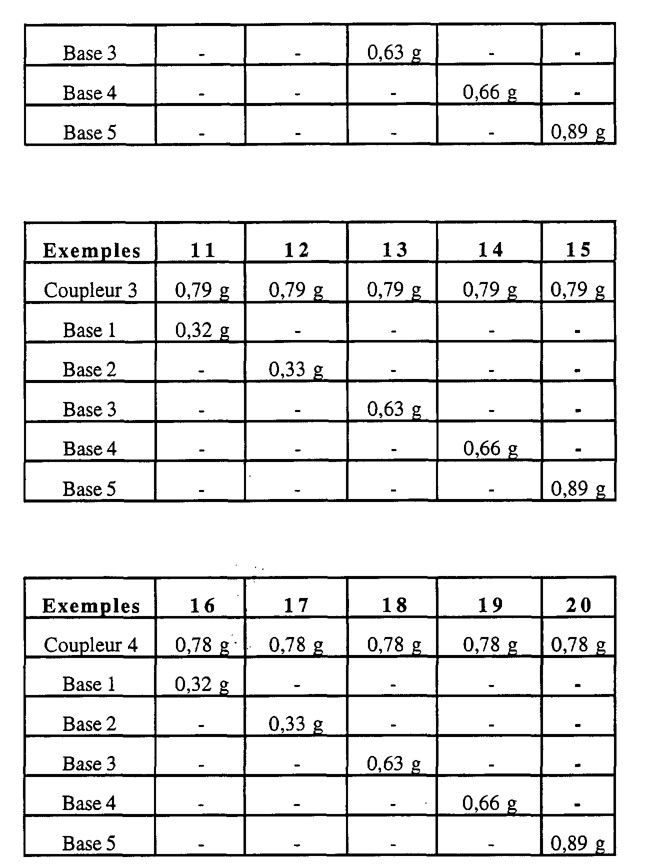

A chacune des compositions tinctoriales ainsi obtenues, on a mélangé une quantité égale d'une composition oxydante constituée par une solution d'eau oxygénée à 20 volumes (6 % en poids) et présentant un pH d'environ 3.

Chaque mélange ainsi obtenu présentait un pH d'environ 9,5 et a été appliqué pendant 30 minutes sur des mèches de cheveux gris naturels à 90 % de blancs. Les mèches de cheveux ont ensuite été rincées, lavées avec un shampooing standard, puis séchées.

On a ensuite déterminé la hauteur de ton et les reflets de la coloration obtenue On obtient alors les résultats suivants :

| **Exemples** | **Coupleur** | **Base** | **Hauteur de ton et Reflets** |
|---|---|---|---|
| 1 | Coupleur 1 | Base 1 | Blond foncé cendré |
| 2 | | Base 2 | Blond très clair doré cendré |
| 3 | | Base 3 | Blond très clair irisé cendré |
| 4 | | Base 4 | Blond foncé violine |
| 5 | | Base 5 | Blond vert bleuté |
| 6 | Coupleur 2 | Base 1 | Blond foncé acajou cendré |
| 7 | | Base 2 | Blond très clair doré cendré |
| 8 | | Base 3 | Blond foncé rouge cendré |
| 9 | | Base 4 | Blond très clair cendré nacré |
| 10 | | Base 5 | Châtain clair cendré intense |
| 11 | Coupleur 3 | Base 1 | Blond foncé acajou nacré |
| 12 | | Base 2 | Blond cuivré nacré cendré |
| 13 | | Base 3 | Blond nacré rouge |
| 14 | | Base 4 | Blond foncé acajou rouge |
| 15 | | Base 5 | Blond cendré bleu mat |
| 16 | Coupleur 4 | Base 1 | Blond foncé cendré mat |
| 17 | | Base 2 | Blond doré nacré |
| 18 | | Base 3 | Blond très clair acajou cendré |
| 19 | | Base 4 | Châtain clair violine |
| 20 | | Base 5 | Châtain clair vert bleuté |

## Revendications

1. Composition pour la teinture d'oxydation des fibres kératiniques, **caractérisée en ce qu'**elle contient, dans un milieu approprié pour la teinture desdites fibres :
- au moins une base d'oxydation;
- et au moins un coupleur choisi parmi les composés de formule (I) suivante et/ou leurs sels d'addition avec un acide : 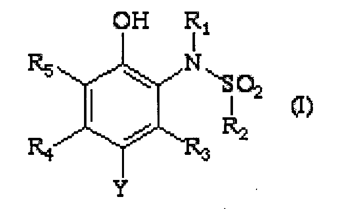
dans laquelle :
• R₁ représente un atome d'hydrogène; un radical comportant de 1 à 15 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), et dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène; ledit radical R₁ ne comportant pas de liaisons peroxyde ni de radicaux diazo, nitro et nitroso;
• R₂ représente un atome d'hydrogène; un radical comportant de 1 à 20 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), et dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène; ledit radical R₂ ne comportant pas de liaisons peroxyde ni de radicaux diazo, nitro et nitroso;
• R₃, R₄ et R₅, identiques ou différents, représentent un atome d'hydrogène ou d'halogène; un radical comportant de 1 à 20 atomes de carbone, linéaire ou ramifié (la ou les ramifications pouvant alors former un ou plusieurs cycles comportant de 3 à 7 chaînons), pouvant contenir une ou plusieurs liaisons doubles et/ou une ou plusieurs liaisons triples (lesdites liaisons doubles conduisant éventuellement à des groupements aromatiques), et dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène; ledit radical ne comportant pas de liaisons peroxydes ni de radicaux diazo, nitro et nitroso; et étant entendu que R₅ ne peut représenter un radical hydroxyle, thio ou amino; et étant entendu que les radicaux R₃, R₄ et R₅ ne peuvent être reliés au cycle benzénique de la formule (I) par une liaison -NH-NH-;
• Y représente un atome d'hydrogène ou d'halogène; un groupement -OR₆, -SR₆ ou -NH-SO₂R₆ dans lesquels R₆ représente un radical alkyle en C₁-C₆, linéaire ou ramifié (la ou les ramifications pouvant alors former un ou plusieurs cycles comportant de 3 à 6 chaînons), éventuellement substitué par un ou plusieurs radicaux choisi dans le groupe : halogène, hydroxy, alcoxy en C₁-C₄, amino, aminoalkyle en C₁-C₄; un radical phényle, éventuellement substitué par un ou deux radicaux choisi dans le groupe alkyle en C₁-C₄, trifluorométhyle, carboxy, alcoxycarbonyle en C₁-C₄, halogène, hydroxy, alcoxy en C₁-C₄, amino, aminoalkyle en C₁-C₄; un radical benzyle.

2. Composition selon la revendication 1, **caractérisée en ce que** dans lesdits composés de formules (I), R₁ désigne un atome d'hydrogène; un groupement A₁ constitué par un radical alkyle en C₁-C₈, linéaire ou ramifié, pouvant porter une ou deux doubles liaisons ou une triple liaison, être substitué ou non substitué par un groupement choisi parmi un groupement A₂, A₄, et A₅ tels que définis ci-après, être substitué ou non substitué par un ou deux groupements, identiques ou différents, choisis parmi les groupements N-alkyl(C₁-C₃)amino, N-alkyl(C₁-C₃)-N-alkyl(C₁-C₃)amino, alcoxy(C₁-C₆), oxo, alcoxycarbonyle, acyloxy, amido, acylamino, uréyle, sulfoxy, sulfonyle, sulfonamido, sulfonylamino, bromo, cyano, carboxy, et être substitué ou non substitué par un ou plusieurs groupements hydroxyle, fluoro ou chloro; un groupement A₂ constitué par un groupement aromatique de type phényle ou naphtyle, pouvant être substitué ou non substitué par un à trois groupements, identiques ou différents, choisis parmi les groupements méthyle, trifluorométhyle, éthyle, isopropyle, butyle, pentyle, fluoro, chloro, bromo, méthoxy, trifluorométhoxy, éthoxy, propyloxy, acétyloxy, acétyle, et cyano; un groupement A₃ constitué par des groupements hétéroaromatiques choisis parmi les groupements furanyle, thiophényle, pyrrolyle, imidazolyle, thiazolyle, oxazolyle, 1,2,3-triazolyle, 1,2,4-triazolyle, isoxazolyle, isothiazolyle, pyrazolyle, pyrazolotriazolyle, pyrazoloimidazolyle, pyrrolotriazolyle, pyrazolopyrimidyle, pyrazolopyridyle, pyridyle, pyrimidyle, benzimidazolyle, benzoxazolyle, benzothiazolyle, indolyle, indolidinyle, isoindolyle, indazolyle, benzotriazolyle, quinolinyle, benzopyrimidyle, substitués éventuellement par 1 à 3 radicaux choisis parmi les alkyle en C₁-C₄ linéaire ou ramifié, (poly)hydroxyalkyle en C₁-C₄, carboxy, alcoxycarbonyle, halogène, amido, amino, hydroxy; un groupement A₄ constitué par un radical cycloalkyle en C₃-C₇, un radical norbornanyle, portant éventuellement une double liaison et éventuellement substitué par 1 ou 2 radicaux définis par alkyle en C₁₋C₄ linéaire ou ramifié, (poly)hydroxyalkyle en C₁-C₄, carboxy, alcoxycarbonyle, halogène, amido, amino, hydroxy; un groupement A₅ constitué par un hétérocycle choisi parmi les cycles dihydrofuranyle, tétrahydrofuranyle, butyrolact-one-yle, dihydrothiophényle, tétrahydrothiophényle, tétrahydrothiophén-one-yle, iminothiolanyle, dihydropyrrolyle, pyrrolidinyle, pyrrolidinone-yle, imidazolidin-one-yle, imidazolidinethione-yle, oxazolidinyle, oxazolidin-oneyle, oxazolanethioneyle, thiazolidinyle, isothiazol-one-yle, mercaptothiazolinyle, pyrazolidin-one-yle, dioxolanyle, pentalactone, dioxanyle, dihydropyridinyle, pipéridinyle, pentalactameyle, morpholinyle, pyrazoli(di)nyle, pyrimi(di)nyle, pyrazinyle, pipérazinyle, et azépinyle.

3. Composition selon la revendication 2, **caractérisée en ce que** R₁ représente un atome d'hydrogène; un radical méthyle, éthyle, isopropyle, allyle, phényle, benzyle, fluorobenzyle, hydroxybenzyle difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle 4-méthylthiobenzyle, 4-méthylsulfonylbenzyle, 4-acétylaminobenzyle, 4-carboxybenzyle, 1-naphtométhyle, 2-naphtométhyle; un groupement 2-hydroxyéthyle, 2-méthoxyéthyle, ou 2-éthoxyéthyle.

4. Composition selon l'une quelconque des revendications 1 à 3, **caractérisée en ce que** dans lesdits composés de formule (I), R₂ désigne un atome d'hydrogène; un groupement amino; un groupement A₁, A₂, A₃, A₄ ou A₅ tels que définis à la revendication 2, lesdits groupements étant éventuellement séparés du soufre, situé en position 8, de la fonction sulfonamide dudit composé de formule (I) par un groupement -NH, ou -N-alkyl(C₁-C₃)-.

5. Composition selon la revendication 4, **caractérisée en ce que** R₂ désigne un radical choisi dans le groupe (G1) constitué par un radical méthyle, trifluorométhyle, éthyle, 2-chloroéthyle, propyle, 3-chloropropyle, isopropyle, butyle, phényle, éthoxy, amino et diméthylamino.

6. Composition selon l'une quelconque des revendications 1 à 5, **caractérisée en ce que** dans lesdits composés de formule (I) R₃ et R₄, identiques ou différents, désignent un atome d'hydrogène ou d'halogène; un groupement hydroxyle ou amino; un groupement A₁, A₄, ou A₅ tels que définis à la revendication 2; un groupement A₁, A₂, A₃, A₄ ou A₅ tels que définis à la revendication 2 et séparés du noyau phénolique de ladite formule (I) par un atome d'oxygène ou par un groupement -NH-, -N-alkyl(C₁-C₃)-, O(CO)-, -NH(CO)-, N-alkyl(C₁-C₃)(CO)-, -NH[C=NH]-, -NH(CO)NH-, -NH(CO)N-alkyl(C₁-C₃)-, -NH(CO)O-, -NHSO₂-, -NHSO₂NH-, ou -NHSO₂₋N-alkyl(C₁-C₃)-.

7. Composition selon la revendication 6, **caractérisée en ce que** R₃ représente un atome d'hydrogène ou de chlore; un radical méthyle, hydroxyméthyle, méthoxyméthyle, 1-hydroxyéthyle, aminométhyle, méthylaminométhyle; un radical hydroxy, méthoxy, acétoxy; un radical amino, méthylamino, 2-hydroxyéthylamino; un groupement -NH(CO)R₇ dans lequel R₇ représente un radical choisi dans le groupe (G2) constitué par les radicaux méthyle, éthyle, propyle, isopropyle, butyle, isobutyle, tert-butyle, pentyle, isopentyle, néopentyle, hexyle; cyclopropyle, cyclobutyle, cyclopentyle, cyclopentylméthyle, 3-cyclopentyl-propyle, cyclohexyle, 2-cyclohexyl-éthyle, norbornan-2-yle, vinyle, 1-méthylvinyle, 2-méthylvinyle, 2,2-diméthylvinyle, allyle, 3-butenyle; phenyle, méthylphényle, diméthylphényle, 2,4,6-triméthylphényle, 4-éthylphényle, (trifluorométhyl)phényle, hydroxyphényle, méthoxyphényle, éthoxyphényle, acétoxyphényle, (trifluorométhoxy)phényle, aminophényle, 4-diméthylaminophényle, fluorophényle, difluorophényle, fluoro(trifluorométhyl)phényle, chlorophényle, dichlorophényle, bromophényle, napht1-yle, napht-2-yle, (2-méthoxy)napht-1-yle, benzyle, 4'-méthoxybenzyle, 2',5'diméthoxybenzyle, 3',4'-diméthoxy-benzyle, 4'-fluorobenzyle, 4'-chlorobenzyle, phénéthyle, 2-phénylvinyle, (1-naphtyl)méthyle, (2-naphtyl)méthyle; tétrahydrofuran2-yle, furan-2-yle, 5-méthyl-2-(trifluorométhyl)furan-3-yle, 2-méthyl-5-phénylfuran-3-yle, thiophén-2-yle (thiophén-2-yl)méthyle, 3-chlorothiophén-2-yle, 2,5-dichlorothiophén-3-yle, benzothiophén-2-yle, 3-chlorobenzothiophén-2-yle, isoxazol-5-yle, 5-méthylisoxazol-3-yle, 3,5-diméthylisoxazol-4-yle, 1,3-diméthylpyrazol-5-yle, 1-éthyl-3-méthylpyrazol-5-yle, 1-tertbutyl-3-méthylpyrazol-5-yle, 3-tertbutyl-1-méthylpyrazol-5-yle, 4-bromo-1-éthyl-3-méthylpyrazol-5-yle, indol-3-ylcarboxyle, pyridinyle, chloropyridinyle, dichloropyridinyle, 5-(bromo)pyridin-3-yle, piperazin-2-yle, quinoxal-2-yle; fluorométhyle, difluorométhyle, trifluorométhyle, 1,1,2,2-tétrafluoroéthyle, pentafluoroéthyle, heptafluoropropyle, 1,1,2,2,3,3,4,4-octafluorobutyle, nonafluorobutyle, chlorométhyle, chloroéthyle, 1,1-diméthyl-2-chloroéthyle, 1,2-dichloroéthyle, 1-chloropropyle, 3-chloropropyle,4-chlorobutyle, hydroxyméthyle, méthoxyméthyle, phénoxyméthyle, (4-chlorophénoxy)méthyle, benzyloxyméthyle, acétoxyméthyle, 1,2-dihydroxyéthyle, 1-phénoxyéthyle, 1-acétoxyéthyle, 2-(2-carboxyéthoxy)éthyle, 1-phénoxyéthyle, 1-acétoxyéthyle, méthoxycarbonyle, éthoxycarbonyle, (méthoxycarbonyl)méthyle, 2-carboxyéthyle, 2-(méthoxycarbonyl)éthyle, 2-carboxycylopropyle, 2-carboxycyclohexane; méthoxy, éthoxy, propoxy, isopropoxy, butoxy, isobutoxy, pentoxy, néopentoxy, hexyloxy, cyclopentyloxy, cyclohexyloxy, vinyloxy, allyloxy, propargyloxy, chlorométhoxy, 1-chloroéthoxy, 2-méthoxyéthoxy, 4-chlorobutoxy, phénoxy, 4-méthyphénoxy, 4-fluorophénoxy, 4-bromophénoxy, 4-chlorophénoxy, 4-méthoxyphénoxy, naphth-2-yloxy, benzyloxy; amino, méthylamino, éthylamino, propylamino, isopropylamino, butylamino, cyclohexylamino, allylamino, 2-chloroéthylamino, 3-chloropropylamino, carboxyméthylamino, phénylamino, fluorophénylamino, (trifluorométhyl)phénylamino, chlorophénylamino, bromophénylamino, 4-acétylphénylamino, méthoxyphénylamino, (trifluorométhoxy)phénylamino, naphth-1-ylamino, benzylamino, phénéthylamino, pyrid-3-ylamino, diméthylamino, 1-pyrolidinyle, et 4-morpholinyle; ou un groupement -NHSO₂R₈, dans lequel R₈ représente un radical choisi dans le groupe (G1) tel que défini à la revendication 5.

8. Composition selon la revendication 6, **caractérisée en ce que** R₄ représente un atome d'hydrogène ou de chlore; un radical méthyle, éthyle, hydroxyméthyle, méthoxyméthyle, aminométhyle, ou méthylaminométhyle; un groupement hydroxy, méthoxy, acétoxy, amino, méthylamino, N-pipéridino, ou N-morpholino; un groupement -NH(CO)R₁₀ dans lequel R₁₀ représente un des radicaux énumérés dans le groupe (G2) tel que défini à la revendication 7; ou un groupement -NHSO₂R₁₁ dans lequel R₁₁ représente un des radicaux énumérés dans le groupe (G1) tel que défini à la revendication 5.

9. Composition selon l'une quelconque des revendications 1 à 8, **caractérisée en ce que** dans lesdits composés de formule (I,) R₅ désigne un atome d'hydrogène ou d'halogène; un groupement A₁, A₄, ou A₅ tels que définis à la revendication 2; un groupement A₁, A₂, A₃, A₄ ou A₅ tels que définis à la revendication 2 et séparés du noyau phénolique des composés de formule (I) par un atome d'oxygène, de soufre, ou par un groupement-NH-, -N-alkyl(C₁-C₃)-, -NH(CO)-, -N-alkyl(C₁-C₃)(CO)-, -NH[C=NH]-, -NH(CO)NH-, -NH(CO)N-alkyl(C₁-C₃)-, ou -NH(CO)O-.

10. Composition selon la revendication 9, **caractérisée en ce que** R₅ représente un atome d'hydrogène, de chlore, de fluor, ou de brome; un radical méthyle, trifluorométhyle, allyle, hydroxyméthyle, méthoxyméthyle, 1-hydroxyéthyle, aminométhyle, méthylaminométhyle, méthoxy, acétoxy, ou méthylamino; un groupement -NH(CO)R₁₃ dans lequel R₁₃ représente un des radicaux énumérés dans le groupe (G2) tel que défini à la revendication 7; ou un groupement -NHSO₂R₁₄ dans lequel R₁₄ représente un des radicaux énumérés dans le groupe (G1) tel que défini à la revendication 5.

11. Composition selon l'une quelconque des revendications 1 à 10, **caractérisée en ce que** dans lesdits composés de formule (I,) Y désigne un atome d'hydrogène, de chlore, de fluor ou de brome; un groupement méthoxy, éthoxy, propoxy, benzyloxy, ou phénoxy; ou un groupement -OCH₂CH₂OCH₃, -OCH₂CH₂N(CH₃)₂, -OCH₂(CO)OH, -OCH₂(CO)OCH₃, -OCH₂(CO)OC₂H₅, -SCH₂CH₂CO₂H, ou -NHSO₂CH₃.

12. Composition selon l'une quelconque des revendications 1 à 11, **caractérisée en ce que** les composés de formule (I) sont choisis parmi ceux dans lesquels :
i)
- R₁ représente un atome d'hydrogène;
- R₂ représente un radical méthyle, éthyle, phényle ou diméthylamino;
- R₃ représente un radical hydroxy, amino, ou méthylamino; un groupement -NH(CO)R₁₆ dans lesquels R₁₆ représente un radical choisi dans le groupe (G4) constitué par les radicaux méthyle, méthoxyméthyle, 2-carboxyéthyle, méthoxy, amino, éthylamino, 1-pyrolidinyle; méthanesulfonylamino, éthanesulfonylamino, et diméthylaminosulfonylamino;
- R₄ représente un atome d'hydrogène ou de chlore; ou un groupement méthyle;
- R₅ représente un atome d'hydrogène, de chlore ou de fluor; ou un groupement méthyle;
- Y représente un atome d'hydrogène ou de chlore; ou un groupement méthoxy, ou -OCH2(CO)OCH3;
ii)
- R₁ représente un atome d'hydrogène;
- R₂ représente un radical méthyle, éthyle, phényle ou diméthylamino;
- R₃ représente un atome d'hydrogène ou un radical méthyle;
- R₄ représente un groupement hydroxy, amino, méthylamino, ou -NH(CO)R₁₇ dans lesquels R₁₇ représente un des radicaux énumérés dans le groupe (G4) défini ci-dessus; un groupement méthanesulfonylamino, éthanesulfonylamino, ou diméthylaminosulfonylamino;
- R₅ représente un atome d'hydrogène, de chlore ou de fluor, ou un groupement méthyle, méthoxy, ou méthylamino;
- Y représente un atome d'hydrogène ou de chlore, ou un groupement méthoxy, ou -OCH₂(CO)OCH₃;
iii)
- R₁ représente un atome d'hydrogène;
- R₂ représente un radical méthyle, éthyle, phényle ou diméthylamino;
- R₃ représente un atome d'hydrogène ou un radical méthyle;
- R₄ représente un atome d'hydrogène ou de chlore, un radical méthyle, méthoxy, ou méthylamino;
- R₅ représente un groupement méthylamino, ou -NH(CO)R₁₈ dans lequel R₁₈ représente un des radicaux énumérés dans le groupe (G4) défini ci-dessus; un groupement méthanesulfonylamino, éthanesulfonylamino, ou diméthylaminosulfonylamino;
- Y représente un atome d'hydrogène ou de chlore; ou un groupement méthoxy, ou -OCH₂(CO)OCH₃;
iv)
- R₁ représente un atome d'hydrogène;
- R₂ représente un radical méthyle, éthyle, phényle ou diméthylamino;
- R₃ représente un atome d'hydrogène ou un radical méthyle;
- R₄ représente un atome d'hydrogène ou de chlore, ou un radical méthyle;
- R₅ représente un atome d'hydrogène, de chlore ou de fluor; ou un radical méthyle;
- Y représente un atome d'hydrogène ou de chlore, ou un groupement méthoxy, ou -OCH₂(CO)OCH₃.

13. Composition selon l'une quelconque des revendications 1 à 12, **caractérisée en ce que** les composés de formule (I) sont choisi parmi :
- le N-(2-hydroxy-phényl)-méthanesulfonamide;
- le N-(2-hydroxy-4-méthyl-phényl)-méthanesulfonamide;
- le N-(2-hydroxy-4-amino-phényl)-méthanesulfonamide;
- le N-(2-hydroxy-4-acétylamino-phényl)-méthanesulfonamide;
- le N-(2-hydroxy-4-méthoxycarbonylamino-phényl)-méthanesulfonamide;
- le N-(2-hydroxy-5-chloro-phényl)-méthanesulfonamide;
- le N-(2-hydroxy-4-méthyl-5-chloro-phényl)-méthanesulfonamide;
- le N-(2-hydroxy-4-amino-5-chloro-phényl)-méthanesulfonamide;
- le N-(2-hydroxy-4-acétylamino-5-chloro-phényl)-méthanesulfonamide;
- le N-(2-hydroxy-4-méthoxycarbonylamino-5-chloro-phényl)-méthanesulfonamide;
- le N-(2-hydroxy-5-méthoxy-phényl)-méthanesulfonamide
- le N-(2-hydroxy-4-méthyl-5-méthoxy-phényl)-méthanesulfonamide;
- le N-(2-hydroxy-4-amino-5-méthoxy-phényl)-méthanesulfonamide;
- le N-(2-hydroxy-4-acétylamino-5-méthoxy-phényl)-méthanesulfonamide;
- le N-(2-hydroxy-4-méthoxycarbonylamino-5-méthoxy-phényl)-méthanesulfonamide;
- le N-(2-hydroxy-6-amino-phényl)-méthanesulfonamide;
- le N-(2-hydroxy-6-acétylamino-phényl)-méthanesulfonamide;
- le N-(2-hydroxy-4,6-diamino-phényl)-méthanesulfonamide;
- le N-(2-hydroxy-4-acétylamino-6-amino-phényl)-méthanesulfonamide;
- le N-(2-hydroxy-3,5-dichloro-4-méthyl-phényl)-méthanesulfonamide;
- le N-(2-hydroxy-3,5-dichloro-4-amino-phényl)-méthanesulfonamide;
- le N-(2-hydroxy-3,5-dichloro-4-acétylamino-phényl)-méthanesulfonamide;
- le N-(2-hydroxy-3,5-dichloro-4-méthoxycarbonylamino-phényl)méthanesulfonamide;
- le N-(2-hydroxy-3-méthanesulfonylamino-phényl)-méthanesulfonamide;
- le N-(2-hydroxy-phényl)-benzènesulfonamide;
- le N-(2-hydroxy-4-méthyl-phényl)-benzènesulfonamide;
- le N-(2-hydroxy-4-amino-phényl)-benzènesulfonamide;
- le N-(2-hydroxy-4-acétylamino-phényl)-benzènesulfonamide;
- le N-(2-hydroxy-4-méthoxycarbonylamino-phényl)-benzènesulfonamide;
- le N-(2-hydroxy-5-chloro-phényl)-benzènesulfonamide;
- le N-(2-hydroxy-4-méthyl-5-chloro-phényl)-benzènesulfonamide;
- le N-(2-hydroxy-4-amino-5-chloro-phényl)-benzènesulfonamide;
- le N-(2-hydroxy-4-acétylamino-5-chloro-phényl)-benzènesulfonamide;
- le N-(2-hydroxy-4-méthoxycarbonylamino-5-chloro-phényl)-benzènesulfonamide;
- le N-(2-hydroxy-5-méthoxy-phényl)-benzènesulfonamide;
- le N-(2-hydroxy-4-méthyl-5-méthoxy-phényl)-benzènesulfonamide;
- le N-(2-hydroxy-4-amino-5-méthoxy-phényl)-benzènesulfonamide;
- le N-(2-hydroxy-4-acétylamino-5-méthoxy-phényl)-benzènesulfonamide;
- le N-(2-hydroxy-4-méthoxycarbonylamino-5-méthoxy-phényl)-benzènesulfonamide;
- le N-(2-hydroxy-6-amino-phényl)-benzènesulfonamide;
- le N-(2-hydroxy-6-acétylamino-phényl)-benzènesulfonamide;
- le N-(2-hydroxy-4,6-diamino-phényl)-benzènesulfonamide;
- le N-(2-hydroxy-4-acétylamino-6-amino-phényl)-benzènesulfonamide;
- le N-(2-hydroxy-3,5-dichloro-4-méthyl-phényl)-benzènesulfonamide;
- le N-(2-hydroxy-3,5-dichloro-4-amino-phényl)-benzènesulfonamide;
- le N-(2-hydroxy-3,5-dichloro-4-acétylamino-phényl)-benzènesulfonamide;
- le N-(2-hydroxy-3,5-dichloro-4-méthoxycarbonylamino-phényl)benzènesulfonamide;
- le N-(2-hydroxy-3-benzènesulfonylamino-phényl)-benzènesulfonamide;
et leurs sels d'addition avec un acide.

14. Composition selon l'une quelconque des revendications 1 à 13, **caractérisée en ce que** le ou les composés de formules (I) et/ou le ou leurs sels d'addition avec un acide représentent de préférence de 0,0005 à 12 % en poids environ du poids total de la composition tinctoriale.

15. Composition selon l'une quelconque des revendications 1 à 14, **caractérisée en ce que** les sels d'addition avec un acide sont choisis parmi les chlorhydrates, les bromhydrates, les sulfates, les citrates, les succinates, les tartrates, les lactates et les acétates.

16. Procédé de teinture des fibres kératiniques et en particulier des fibres kératiniques humaines telles que les cheveux **caractérisé en ce que** l'on applique sur lesdites fibres au moins une composition tinctoriale telle que définie à l'une des revendications 1 à 15, et **en ce que** l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

17. Procédé selon la revendication 16, **caractérisé en ce que** l'agent oxydant est choisi parmi le peroxyde d'hydrogène, le peroxyde d'urée, les bromates de métaux alcalins, les persels et les enzymes.

18. Procédé selon la revendication 17, **caractérisé en ce que** les enzymes sont choisies parmi les peroxydases, les laccases, les tyrosinases et les oxydo-réductases.

19. Dispositif à plusieurs compartiments, ou "kit" de teinture à plusieurs compartiments dont un premier compartiment renferme une composition tinctoriale telle que définie à l'une quelconque des revendications 1 à 15 et un second compartiment renferme une composition oxydante
